# FASCICULE DE BREVET EUROPEEN

(11) **EP 1 185 655 B1**
(45) Date de publication et mention de la délivrance du brevet: **30.09.2009**
(21) Numéro de dépôt: 00940472.4
(22) Date de dépôt: 08.06.2000
(51) Int. Cl.: C12N 15/27, C07K 14/535, C12N 15/62, A61K 39/39, A61K 48/00

(54) **GM-CSF EQUIN**
GM-CSF AUS PFERD
EQUINE GRANULOCYTE-MACROPHAGE COLONY-STIMULATING FACTOR (GM-CSF)

(30) Priorité: 10.06.1999 US 138843 P
(43) Date de publication de la demande: 13.03.2002
(73) Titulaire: MERIAL, 69007 Lyon (FR)
(72) Inventeur: BUBLOT, Michel, Delmar, NY 12054 (US); PEREZ, Jennifer, Maria, Worcester, MA 01605-1491 (US); ANDREONI, Christine, Michèle, Pierrette, F-38280 Villette d'Anthon (FR)
(74) Mandataire: Nargolwalla, Cyra
(86) Numéro de dépôt international: PCT/FR2000/001590
(87) Numéro de publication internationale: WO 2000/077210

(56) Documents cités:
- WO-A-92/05255
- WO-A-94/01133
- WO-A-98/03198
- DATABASE EMBL [en ligne] CE14392, accession number U14392, 14 septembre 1994 (1994-09-14) E.A. LOCKHART : "Cloning and sequencing of cervine GM-CSF" XP002148816 & UNPUBLISHED,
- MCINNES C J ET AL: "CLONING AND EXPRESSION OF A CDNA ENCODING OVINE GRANULOCYTE-MACROPHAGE COLONY-STIMULATING FACTOR" GENE (AMSTERDAM), vol. 105, no. 2, 1991, pages 275-279, XP002148815 ISSN: 0378-1119
- INUMARU S ET AL: "CDNA cloning of porcine granulocyte-macrophage colony-stimulating factor." IMMUNOLOGY AND CELL BIOLOGY, vol. 73, no. 5, 1995, pages 474-476, XP000946635 ISSN: 0818-9641
- SIN J -I ET AL: "Protective immunity against heterologous challenge with encephalomyocarditis virus by VP1 DNA vaccination: effect of coinjection with a granulocyte-macrophage colony stimulating factor gene" VACCINE,GB,BUTTERWORTH SCIENTIFIC. GUILDFORD, vol. 15, no. 17-18, 1 décembre 1997 (1997-12-01), pages 1827-1833, XP004097373 ISSN: 0264-410X
- HARTIKKA J: "AN IMPROVED PLASMID DNA EXPRESSION VECTOR FOR DIRECT INJECTION INTOSKELETAL MUSCLE" HUMAN GENE THERAPY,XX,XX, vol. 7, no. 10, 20 juin 1996 (1996-06-20), pages 1205-1217, XP002050079 ISSN: 1043-0342 cité dans la demande

## Description

La présente invention a trait à la séquence nucléotidique du gène codant pour la cytokine GM-CSF du cheval , à des vecteurs d'expression la contenant, et à son utilisation comme adjuvant dans la vaccination équine et comme stimulant non spécifique de l'immunité.

La première découverte d'un facteur de stimulation de la formation de colonies granulo-macrophagiques (en anglais Granulocyte-Macrophage Colony-Stimulating Factor ou GM-CSF) date de 1977 (Burgess A. W. et al. J. Biol. Chem. 1977. 252. 1998-2003). Il s'agit du GM-CSF murin, purifié à partir de surnageants de culture de poumons de souris.

Les activités biologiques du GM-CSF ont été mises en évidence par des travaux sur les GM-CSF murin et humain (Clark S. C. et al. Science 1987. 230. 1229 ; Grant S. M. et al. Drugs 1992. 53. 516).

Le GM-CSF a de nombreux rôles physiologiques (Dy M. dans "Les cytokines" Cavaillon J.-M., 1996, éd. Masson, Paris, France, 43-56). En particulier, le GM-CSF stimule la production, le développement, et la formation de colonies de granulocytes, de macrophages, d'éosinophiles et de mégacaryocytes. Le GM-CSF induit en particulier une cytotoxicité macrophagique, stimule l'activité cytotoxique anticorps-dépendante (ADCC) et le recrutement des leucocytes au niveau des sites d'inflammation.

Les GM-CSF de différentes espèces animales ont déjà été mis en évidence.

Les tailles des séquences nucléotidiques codant pour les GM-CSF connues de différentes espèces varient de 381 à 432 nucléotides. Les séquences nucléotidiques humaines et murines ont un degré d'homologie de 69 %. Le degré d'homologie est de 54 % au niveau séquence en acides aminés (Cantrell M. A. et al. Proc. Natl. Acad. Sci. USA 1985. 82. 6250-6254). Cependant cette homologie ne permet aucune activité croisée entre les deux espèces humaine et murine (Metcalf D. et al. Blood 1986. 67. 37-45).

L'administration de GM-CSF hétérologue, c'est-à-dire provenant d'une espèce autre que celle traitée, ne permet pas d'obtenir un effet adjuvant optimal, notamment une stimulation de l'activité des cellules hématopoïétiques et une augmentation substantielle de la réponse immunitaire.

Jusqu'à ce jour, le GM-CSF équin n'a pas pu être mis en évidence. Or cette cytokine présente un intérêt important pour les applications thérapeutiques et vaccinales à l'usage des chevaux.

La déposante a réussi à isoler et séquencer le gène du GM-CSF équin. Ce gène a été isolé après amplification en chaîne par polymérase (ACP ou PCR) avec l'aide des oligonucléotides décrits dans les exemples.

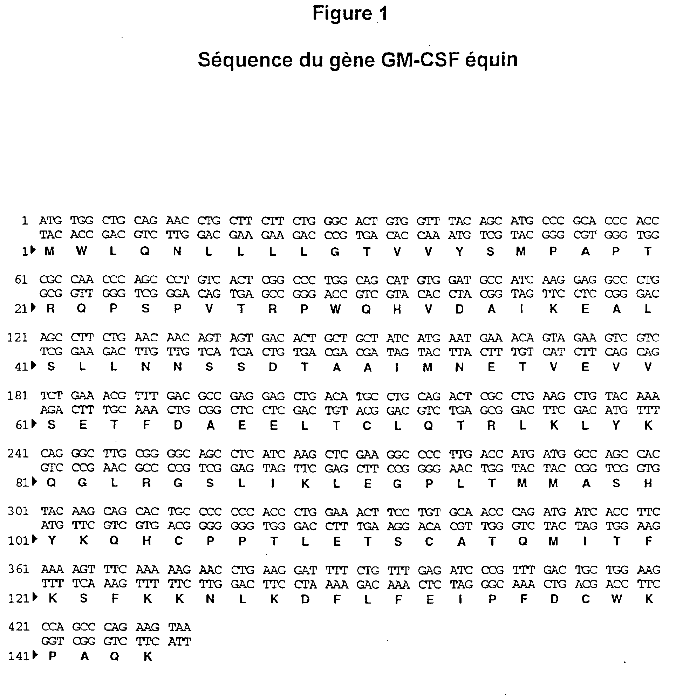

Le gène GM-CSF équin a une taille de 432 nucléotides (SEQ ID N° 8 et Figure 1) et code pour une protéine de 144 acides aminés (SEQ ID N° 9 et Figure 1). La protéine codée par ce gène présente une homologie d'au moins 75 % avec les séquences polypeptidiques GM-CSF d'autres espèces animales.

La présente invention a donc pour objet un fragment d'ADN isolé codant pour le GM-CSF équin, comprenant la SEQ ID N°8, Elle a aussi pour objet le fragment d'ADN ayant, ou consistant essentiellement en, cette séquence.

La présente invention a aussi pour objet un fragment d'ADN isolé codant pour la séquence en acides aminés SEQ ID N°9.

Elle a aussi pour objet des fragments d'ADN comprenant une séquence nucléotidique codant pour le GM-CSF équin, selon SEQ ID N°8 ou une séquence codant pour la séquence en acides aminés SEQ ID N°9, cette séquence nucléotidique étant associée, en fusion, avec la séquence nucléotidique codant pour au moins un immunogène ou au moins un fragment immunologiquement actif ou au moins un épitope d'un immunogène. Le fragment d'ADN ne comporte alors pas de codon stop entre la séquence codant pour le GM-CSF et la séquence d'immunogène associée. Par exemple, si l'on se réfère à la SEQ ID N°8, la séquence codante insérée se termine au nucléotide 432, et n'inclut pas le codon stop.

La présente invention a également pour objet la protéine ou polypeptide GM-CSF équin isolé, tel que codé par la séquence nucléotidique SEQ ID N° 8.

La présente invention a aussi pour objet la protéine GM-CSF équin ayant la séquence en acides aminés SEQ ID N° 9.

La protéine GM-CSF équin a une taille de 144 acides aminés. La présente invention comprend également les protéines de fusion, tant que leur activité biologique (pour la partie commune avec GM-CSF) est équivalente à celle de la protéine GM-CSF équin naturelle *in vivo* chez le cheval et que leur spécificité d'espèce n'est pas modifiée. Sont comprises comme équivalents les séquences en acide aminés codées par n'importe laquelle des séquences nucléotidiques équivalentes définies plus haut.

La présente invention a aussi pour objet une préparation pure de protéine GM-CSF équin.

La présente invention a aussi pour objet les vecteurs d'expression comportant comme insert l'un des fragments d'ADN ou séquences nucléotidiques définies plus haut, en particulier le gène GM-CSF équin (SEQ ID N° 8) ou un équivalent de celui-ci tel que défini précédemment, ainsi que toute séquence nucléotidique codant pour toute séquence d'acides aminés telle que définie plus haut. De même, le vecteur peut en outre comprendre une séquence nucléotidique codant pour au moins un immunogène ou au moins un fragment immunologiquement actif ou au moins un épitope d'un immunogène, qui peut être
ou non associée en fusion comme décrit ci-dessus.

La séquence nucléotidique peut être insérée dans des systèmes d'expression *in vitro* classiques, d'origine virale telle que Baculovirus, notamment propagé sur cellules d'insectes, ou des cellules d'origine procaryote (par exemple *Escherichia coli*) ou eucaryote, en particulier des levures, notamment *Saccharomyces cerevisiae*, des cellules eucaryotes de mammifères, notamment des cellules de hamster (par exemple les cellules ovariennes d'hamster ou CHO) et des cellules de chevaux. L'invention couvre donc également ces systèmes d'expression transformés par une séquence selon l'invention, les protéines GM-CSF équin ainsi produites, et leur usage en tant qu'adjuvant de vaccin et de stimulant non spécifique de l'immunité.

De manière préférée, la séquence selon l'invention est introduite dans des vecteurs d'expression *in vivo* dans des conditions permettant l'expression chez le cheval d'une protéine GM-CSF équin fonctionnelle, et éventuellement une séquence nucléotidique codant pour au moins un immunogène ou au moins un fragment immunologiquement actif ou au moins un épitope d'un immunogène. Ces vecteurs d'expression peuvent être des plasmides, des vecteurs viraux, tels que les poxvirus, par exemple le virus de la vaccine, les avipox (canarypox, folwpox), y compris les poxvirus spécifiques d'espèce (swine pox, raccoonpox et camelpox), les adénovirus et les herpèsvirus, tels que les virus herpès équins.

Le terme plasmide entend recouvrir toute unité de transcription ADN sous forme d'une séquence polynucléotidique comprenant la séquence du gène GM-CSF équin et les éléments nécessaires à son expression *in vivo.* On préfére la forme plasmide circulaire, superenroulée ou non. La forme linéaire entre également dans le cadre de cette invention.

Chaque plasmide comprend un promoteur apte à assurer, dans les cellules hôtes, l'expression du gène inséré sous sa dépendance. Il s'agit en général d'un promoteur eucaryote fort et en particulier d'un promoteur précoce du cytomégalovirus CMV-IE, d'origine humaine ou murine, ou encore éventuellement d'une autre origine telle que rat, cobaye. De manière plus générale, le promoteur est soit d'origine virale, soit d'origine cellulaire. Comme promoteur viral autre que CMV-IE, on peut citer le promoteur précoce ou tardif du virus SV40 ou le promoteur LTR du virus du Sarcome de Rous. Il peut aussi s'agir d'un promoteur de virus dont provient le gène, par exemple le promoteur propre du gène. Comme promoteur cellulaire, on peut citer le promoteur d'un gène du cytosquelette, tel que par exemple le promoteur de la desmine, ou encore le promoteur de l'actine. Lorsque plusieurs gènes sont présents dans le même plasmide, ceux-ci peuvent être présentés dans la même unité de transcription ou dans deux unités différentes.

Les plasmides peuvent également comprendre d'autres éléments de régulation de transcription, tels que par exemple des séquences stabilisatrices du type intron, de préférence intron Il du gène de la β-globine de lapin (van Ooyen et al. Science, 1979, 206: 337-344), séquence signal de la protéine codée par le gène de l'activateur du plasminogène tissulaire (tPA ; Montgomery et al. Cell. Mol. Biol. 1997, 43: 285-292), et signal de polyadénylation (polyA), notamment du gène de l'hormone de croissance bovine (bGH) (US-A-5 122 458) ou du gène de la β-globine du lapin.

L'invention couvre également les compositions immunogènes et les vaccins comprenant la protéine GM-CSF équin selon l'invention, et au moins une préparation immunogène ou vaccinale de pathogène équin, et un excipient ou véhicule acceptable sur le plan vétérinaire. La notion de préparation immunogène recouvre ici toute préparation susceptible, une fois administrée au cheval, d'induire une réponse immunitaire dirigée contre le pathogène équin considéré, réponse qui est augmentée par la présence de la protéine GM-CSF. Il s'agit de préférence d'une préparation vaccinale capable d'induire une protection efficace ou un certain degré de protection contre ce pathogène, degré de protection qui est ici accru par la présence de la protéine GM-CSF équin. Les préparations immunogènes et vaccinales visées dans l'invention recouvrent tous les types connus, tels que préparations inactivées, vivantes atténuées, de sous-unités et recombinantes (utilisant un vecteur d'expression *in vivo,* notamment d'origine virale ou plasmidique). Comme on l'a vu plus haut, la protéine GM-CSF peut être ajoutée telle quelle à la préparation immunogène ou vaccinale pour former, en présence d'un excipient ou véhicule acceptable sur le plan vétérinaire, une composition immunogène ou un vaccin prêt à être administré. On peut aussi prévoir de combiner la protéine GM-CSF à un système de libération prolongée conçu pour libérer graduellement la protéine.

Suivant une modalité plus avantageuse de l'invention, on préfère cependant exprimer la protéine GM-CSF *in vivo* en utilisant un vecteur d'expression *in vivo* tel que décrit ci-dessus. Dans ce cas, on préfère aussi que la préparation immunogène
ou vaccinale soit aussi de type recombinant, basée sur l'utilisation d'un vecteur d'expression *in vivo,* de même type ou de type différent. On peut aussi prévoir d'utiliser un même vecteur d'expression *in vivo,* comprenant et exprimant au moins un immunogène de pathogène équin et la protéine GM-CSF équin.

Les avantages de l'utilisation de GM-CSF lors des vaccinations sont notamment la diminution de la dose d'immunogène ou de vecteur ou d'ADN utilisé. De plus, chez certains animaux non répondeurs lors de l'administration d'un vaccin usuel, l'utilisation du GM-CSF permet la stimulation de la réponse immunitaire et son augmentation jusqu'à un niveau protecteur.

La présente invention couvre donc de préférence les compositions immunogènes et les vaccins comprenant :
- un vecteur d'expression *in vivo* contenant une séquence nucléotidique codant pour un GM-CSF équin, dans des conditions permettant l'expression chez le cheval d'une protéine GM-CSF équin fonctionnelle,
- au moins un vecteur d'expression *in vivo* contenant au moins une séquence nucléotidique codant pour au moins un immunogène équin, étant entendu que ce vecteur ou certains ou tous ces vecteurs (lorsqu'il y a plusieurs vecteurs codant pour des immunogènes différents) peuvent constituer aussi le vecteur GM-CSF (le vecteur comprend au moins une séquence GM-CSF et une séquence d'immunogène), et
- un véhicule ou excipient acceptable sur le plan vétérinaire.

Suivant la modalité préférée de l'invention, l'invention couvre les compositions immunogènes et les vaccins de type ADN, comprenant un plasmide codant et exprimant le GM-CSF équin selon l'invention et au moins un autre plasmide codant et exprimant un immunogène équin ou un fragment immunologiquement actif dérivé de ce dernier. Des exemples de constructions de plasmides, utilisables dans l'invention, contenant un immunogène équin sont donnés dans la demande de brevet WO-A-9803198. L'invention couvre également les vaccins ADN comprenant un plasmide codant et exprimant simultanément le GM-CSF équin et au moins un immunogène équin.

L'invention vise tous les pathogènes équins. On peut citer plus particulièrement : virus herpès équin de type 1 ou de type 4 (et de préférence l'invention prévoit la combinaison deys deux types), virus de la grippe équine, tétanos, *Borrelia burgdorferi*, encéphalites équines de l'Est, de l'Ouest, du Venezuela, virus de la rage. Pour les vaccins de sous-unités et les vaccins recombinants, les immunogènes équins sont de préférence choisis parmi le groupe comprenant les glycoprotéines gB, gC, gD du virus herpès équin de type 1 ou de type 4, l'hémagglutinine (HA) et la nucléoprotéine (NP) du virus de la grippe équine, le fragment sous-unitaire C de la toxine tétanique, la protéine OspA de *Borrelia burgdorferi*, les gènes E2 et C des encéphalites équines de l'Est, de l'Ouest, du Venezuela, le gène G du virus de la rage.

La présente invention a encore pour objet des compositions stimulantes non spécifiques de l'immunité, c'est-à-dire utilisables comme stimulant général de l'immunité chez le cheval. Ces compositions sont administrées en présence comme en l'absence de pathologie déclarée, en général indépendamment de tout vaccin, afin de renforcer les défenses immunitaires du cheval. Ces compositions comprennent du GM-CSF selon l'invention, sous toutes les formes décrites ci-dessus, protéine ou recombinante, de préférence recombinante (vecteur d'expression *in vivo* viral ou plasmidique), et un excipient ou véhicule acceptable sur le plan vétérinaire. Les caractéristiques de ces vecteurs ont déjà été décrites.

Les compositions stimulantes non spécifiques et les compositions immunogènes et les vaccin selon l'invention peuvent aussi comprendre un ou plusieurs adjuvants de l'immunité, notamment choisis parmi ceux habituellement utilisés en vaccination équine contre le ou les pathogènes (valences) considérés. Les compositions stimulantes et les compositions immunogènes et vaccins classiques (inactivés, vivants atténués, sous-unités) peuvent ainsi comprendre à titre d'adjuvant classique des composés de type carbomer, de l'hydroxyde d'alumine, ou être formulés sous forme d'émulsion huile-dans-l'eau. Pour les compositions stimulantes et les compositions immunogènes et vaccins recombinants à base de vecteur d'expression viral, on peut citer les émulsions huile-dans-l'eau.

Suivant une modalité préférée de l'invention, pour les compositions stimulantes de type plasmidique et les composition immunogènes et vaccins de type plasmidique, le plasmide codant et exprimant le GM-CSF équin, le plasmide codant et exprimant GM-CSF et au moins un immunogène équin, ainsi que les mélanges de plasmides contenant ce dernier et au moins un plasmide codant pour un immunogène équin, peuvent être avantageusement formulés de manière originale avec un lipide cationique contenant un sel d'ammonium quaternaire, de formule : 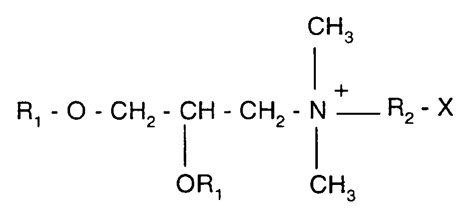 dans laquelle R₁ est un radical aliphatique linéaire, saturé ou insaturé, ayant 12 à 18 atomes de carbone, R₂ est un autre radical aliphatique, renfermant 2 ou 3 atomes de carbone, et X un groupement hydroxyle ou amine.

De préférence il s'agit du DMRIE (N - (2 - hydroxyéthyl) - N , N - diméthyl - 2,3 - bis (tetradécyloxy) - 1 -propanammonium ; WO-A-9634109), de préférence couplé avec un lipide neutre, le DOPE (dioléoyl-phosphatidyl-éthanolamine), pour former préférentiellement le DMRIE-DOPE. De préférence, le mélange vecteur recombinant avec cet adjuvant se fait de manière extemporanée et l'on préfère, avant son administration à l'animal, laisser le temps au mélange ainsi constitué de se complexer, par exemple pendant une durée allant de 10 à 60 minutes, notamment de l'ordre de 30 minutes.

Lorsque du DOPE est présent, le ratio molaire DMRIE : DOPE va de préférence de 95 : 5 à 5 : 95, plus particulièrement de 1 : 1.

Le ratio pondéral plasmide : adjuvant DMRIE ou DMRIE-DOPE peut aller notamment de 50 : 1 à 1 : 10, notamment de 10 : 1 à 1 : 5, de préférence de 1 : 1 à 1 : 2.

Suivant une autre modalité avantageuse de l'invention, pour les compositions stimulantes de type recombinant et les composition immunogènes et vaccins de type recombinant (vecteur viral ou plasmide), on peut utiliser à titre d'adjuvant des polymères de l'acide acrylique ou méthacrylique ou des copolymères d'anhydride maléique et de dérivé alcényle. On préfère les polymères de l'acide acrylique ou méthacrylique réticulés, notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 (incorporé par référence) décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'allylpentaérythritol.
Parmi eux, on peut citer les Carbopol® 974P, 934P et 971 P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields et al., Nature, 186: 778-780, 4 juin 1960 (incorporé par référence). Sur le plan de leur structure, les polymères d'acide acrylique ou méthacrylique et les EMA® sont formés de préférence de motifs de base de formule suivante : 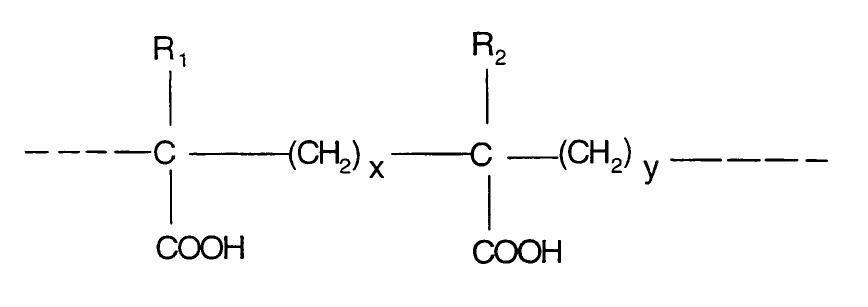 dans laquelle :
- R₁ et R₂, identiques ou différents, représentent H ou CH₃
- x = 0 ou 1, de préférence x = 1
- y = 1 ou 2, avec x + y = 2

Pour les EMA®, x = 0 et y = 2. Pour les carbomères, x = y = 1.

La dissolution de ces polymères dans l'eau conduit à une solution acide qui sera neutralisée, de préférence jusqu'à pH physiologique, pour donner la solution adjuvante dans laquelle le vaccin proprement dit sera incorporé. Les groupes carboxyliques du polymère sont alors en partie sous forme COO⁻.

De manière préférée, on réalise une solution de carbomère ou d'EMA®, dans de l'eau distillée, de préférence en présence de chlorure de sodium, la solution obtenue étant à pH acide. On dilue cette solution mère en l'ajoutant dans la quantité nécessaire (pour l'obtention de la concentration finale souhaitée), ou une partie importante de celle-ci, d'eau chargée en NaCl, de préférence eau physiologique (NaCl 9 g/l), en une ou plusieurs fois avec neutralisation concomitante ou subséquente (pH 7,3 à 7,4), de préférence par NaOH. Cette solution à pH physiologique sera utilisée telle quelle pour mélange avec la préparation immunogène ou vaccinale, notamment conservée sous forme lyophilisée, liquide ou congelée.

La concentration en polymère dans la composition vaccinale finale sera de 0,01 % à 2 % P/V, plus particulièrement de 0,06 à 1 % P/V, de préférence de 0,1 à 0,6 % P/V.

Un autre objet de l'invention est une méthode de stimulation de l'immunité et/ou d'immunisation et/ou de vaccination de l'espèce équine, dans lequel on administre à un animal de l'espèce équine, en particulier un cheval, une composition stimulante, immunogène et/ou vaccinale selon l'invention. L'administration est de préférence réalisée par la voie parentérale, telle que la voie intramusculaire, intradermique ou sous-cutanée. Une ou plusieurs administrations peuvent être réalisées. En particulier, en cas de vaccination, une administration est réalisée à chaque fois que le vaccin est administré.

La quantité d'ADN utilisée dans les compositions stimulantes et les compositions immunogènes et vaccins selon la présente invention est comprise entre environ 10 µg et environ 2000 µg, et préférentiellement entre environ 50 µg et environ 1000 µg, pour un plasmide donné. L'homme de l'art possède les compétences nécessaires pour définir précisément la dose efficace d'ADN à utiliser pour chaque protocole thérapeutique ou de vaccination.

Si un vecteur vivant est utilisé, les doses peuvent être comprises entre 10⁴ et 10¹⁰ ufp (unité formatrice de plaque), de préférence entre 10⁶ et 10⁸ pfu.

Pour une composition contenant la protéine GM-CSF, les doses peuvent être comprises entre 1 µg et 5 mg, de préférence entre 50 µg et 1 mg.

Les volumes de dose peuvent être notamment compris entre 0,5 et 5 ml, de préférence entre 2 et 3 ml.

L'invention va être maintenant décrite plus en détail à l'aide de modes de réalisation pris à titre d'exemples non limitatifs et se référant au dessin dans lequel :
**Figure 1** : Séquences du gène et de la protéine GM-CSF équin
**Figure 2** **:** Carte de restriction du plasmide pJP097

**Liste des séquences SEQ ID pour les constructions de la présente invention**
**SEQ ID N° 1** Oligonucléotide JP705
**SEQ ID N° 2** Oligonucléotide JP706
**SEQ ID N° 3** Oligonucléotide JP729
**SEQ ID N° 4** Oligonucléotide JP730
**SEQ ID N° 5** Oligonucléotide JP731
**SEQ ID N° 6** Oligonucléotide JP734
**SEQ ID N° 7** Oligonucléotide JP735
**SEQ ID N° 8** Séquence du gène GM-CSF équin (voir figure 1)
**SEQ ID N° 9** Séquence de la protéine GM-CSF du cheval (voir figure 1)

### EXEMPLES

Toutes les constructions de plasmides ont été réalisées en utilisant les techniques standards de biologie moléculaire (clonage, digestion par les enzymes de restriction, synthèse d'un ADN complémentaire simple brin, amplification en chaîne par polymérase, élongation d'un oligonucléotide par une ADN polymérase...) décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention, ainsi que les divers fragments d'amplification en chaîne par polymérase (= ACP ou PCR), ont été isolés et purifiés en utilisant le kit "Geneclean®" (BIO101 Inc. La Jolla, CA).

### Exemple 1 : Préparation de l'ARN total de lymphocytes de cheval stimulés in vitro par des mitogènes

Du sang de cheval a été récolté sur un tube contenant de l'EDTA par une prise de sang à la veine jugulaire. Les cellules mononucléées ont été récoltées par centrifugation sur un gradient de Ficoll, puis mises en culture en boîte de Petri de 60 mm de diamètre. Les cellules mononuclées de cheval en culture ont alors été stimulés soit avec de la concanavaline A (conA) (concentration finale d'environ 5 µg/ml) soit avec de la phyto-hémagglutinine (PHA) (concentration finale d'environ 10 µg/ml). Après stimulation, les lymphoblastes « ConA » et « PHA » ont été récoltés par grattage des boîtes de culture, et l'ARN total de ces cellules a été extrait en utilisant le kit « mRNA isolation kit for White Blood Cells » (Boehringer Mannheim/Roche Cat # 1 934 325).

### Exemple 2 : Isolement du gène codant pour le GM-CSF équin

Les oligonucléotides JP075 et JP076 ont été synthétisés et ont les séquences suivantes :
JP705 (SEQ ID N° 1) (20 mer)
   5' TGGGCACTGTGGYCTGCAGC 3'
JP706 (SEQ ID N° 2) (17 mer)
   5' AGCATGTGRATGCCATC 3'

Ces oligonucléotides ont été utilisés avec le kit 5'/3'RACE (Boehringer Mannheim/Roche Cat # 1 734 792) pour générer les clones 3'RACE 6S4, 6W6 et 6W7. La séquence consensus 3' établie à partir de ces 3 clones a été utilisée pour synthétiser les oligonucléotides JP729, JP730 et JP731 devant servir à la génération des clones 5'RACE correspondants :
JP729 (SEQ ID N° 3) (21 mer)
   5' AGCTCCCAGGGCTAGCTCCTA 3'
JP730 (SEQ ID N° 4) (21 mer)
   5' CCCTGTTTGTACAGCTTCAGG 3'
JP731 (SEQ ID N° 5) (21 mer)
   5' TGTTGTTCAGAAGGCTCAGGG 3'

Les clones 5'RACE correspondants obtenus ont été les clones 7D2 et 7D10. Les séquences consensus générées à partir des clones 3'RACE et des clones 5'RACE ont été utilisées pour amplifier la totalité de la séquence du gène GM-CSF équin selon la technique de réverse transcriptase suivie d'une ACP. L'ARN total extrait des lymphocytes de cheval stimulés par la ConA ou par la PHA (exemple 1) a servi de matrice pour la synthèse du premier brin d'ADN complémentaire. Ce premier brin d'ADN complémentaire a été produit par élongation de l'oligonucléotide p(dT)15 (Boehringer Mannheim/Roche Cat # 814 270). L'ADN complémentaire simple brin obtenu a été ensuite utilisé comme matrice pour une réaction d'ACP avec les oligonucléotides suivants :
JP734 (SEQ ID N° 6) (44 mer)
   5' CATCATCATGTCGACGCCACCATGTGGCTGCAGAACCTGCTTCT 3'
et JP735 (SEQ ID N°7) (41 mer)
   5' CATCATCATGCGGCCGCTACTTCTGGGCTGCTGGCTTCCAG 3'
pour amplifier un fragment ACP d'environ 500 paires de bases (pb). Ce fragment a été purifié par électrophorèse en gel d'agarose (= fragment A).

### Exemple 3 : Construction du plasmide pJP097 et séquence du gène GM-CSF équin

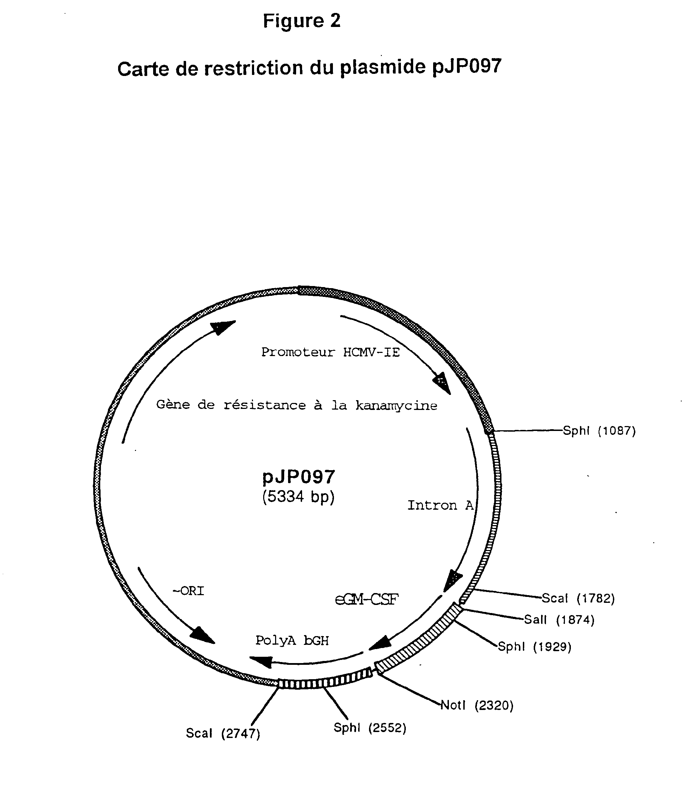

Le fragment A (exemple 2) a été digéré par Notl et Sall et le fragment Notl-Sall ainsi obtenu a été ligaturé avec le plasmide pVR1012 (Hartikka J. et al. Human Gene Therapy. 1996. 7. 1205-1217), préalablement digéré avec Notl et Sall, pour donner le plasmide pJP097 (5334 pb, figure 2). Le fragment Notl-Sall cloné sur ce plasmide a été entièrement séquencé. Cette séquence (SEQ ID N° 8), qui code pour une protéine de 144 acides aminés (SEQ ID N° 9) est la cytokine GM-CSF du cheval (= GM-CSF équin) représentée sur la figure 1.

### Exemple 4 : Activité biologique in vitro du produit du gène GM-CSF équin

Des cellules CHO-K1 (cellules ovariennes de hamster, accessible auprès de la souchothèque American Type Culture Collection sous le numéro d'accès CCL-61) sont mises en culture en milieu essentiel minimum ou MEM (Gibco-BRL) dans des boîtes de Petri de 60 mm de diamètre et transfectées avec 5 µg plasmide pJP097, préalablement complexés avec 10 µl de LipofectAmine PLUS® (Cat# 10964-013, Gibco-BRL, Cleveland, OH, USA). Les conditions de formation des complexes ADN/LipofectAmine® et de transfection des cellules ont été celles recommandées par le fournisseur (Gibco-BRL). 48 heures après la transfection, les surnageants des cultures sont récoltés et congelés.

Des cellules de moelle osseuse prélevées sur des porcs sont mises en culture dans un milieu semi-solide Methocult (Cat# H4230 de StemCell Technologies). Ces cultures sont alors additionnées ou non (contrôle négatif) avec 10 µl du surnageant des cellules transfectées avec le plasmide pJP097. Deux transfections indépendantes ont été réalisées avec le plasmide pJP097, codées pJP097 T1 et pJP097 T2. Chaque surnageant (10 µl dilués au dixième) est testé en parallèle sur 3 boîtes de culture. Le contrôle négatif est constitué par un surnageant de culture CHO. Après 14 jours de culture, les boîtes sont examinées pour la formation de colonies de macrophages, et les éventuelles colonies sont comptabilisées.

Les surnageants de cellules CHO transfectées avec le plasmide pJP097 ont donné les résultats suivants :

| Plasmide / Dilution surnageant | nb de boîtes | nombre moyen de colonies par boîte | Ecart-type |
|---|---|---|---|
| témoin | 3 | 0 | 0 |
| pJP097 T1 (eGM-CSF) | 3 | 12 | 2 |
| pJP097 T2 (eGM-CSF) | 3 | 15 | 0 |

Ces résultats montrent que le produit du gène GM-CSF équin exprimé par le plasmide pJP097 possède une activité de type GM-CSF sur cellules *in vitro.*

### Exemple 5 : Préparation des plasmides selon l'invention

Pour la préparation des plasmides destinés à la vaccination des chevaux, on peut utiliser toute technique permettant d'obtenir une suspension de plasmides purifiés. Ces techniques sont bien connues de l'homme du métier. La production des plasmides se fait par culture de bactéries *Escherichia coli* K12 transformées avec les plasmides selon l'invention. On peut citer en particulier la technique de lyse alcaline suivie de deux ultracentrifugations successives sur gradient de chlorure de césium en présence de bromure d'éthidium telle que décrite dans Sambrook J. et al. (Molecular Cloning : A Laboratory Manual. 2nd edition. Cold Spring Harbor Laboratory. Cold Spring Harbor. NY. 1989). On peut se référer également aux demandes de brevet WO-A-95/21250 et WO-A-96/02658 qui décrivent des méthodes pour produire à l'échelle industrielle des plasmides utilisables pour la vaccination. Pour les besoins de la fabrication des vaccins, les plasmides sont resuspendus de manière à obtenir des solutions à haute concentration (> 2 mg/ml) compatibles avec le stockage. Pour ce faire, les plasmides sont resuspendus soit en eau ultrapure, soit en tampon TE (Tris-HCl 10 mM ; EDTA 1 mM ; pH 8.0).

### Exemple 6 : Fabrication des vaccins selon l'invention et administration

Le stock de plasmide pJP097 est dilué en tampon TE, en eau physiologique ou en tampon PBS, et mélangé à divers plasmides vaccinaux exprimant des immunogènes protecteurs. Ces plasmides peuvent être, par exemple, ceux cités dans les exemples de la demande de brevet PCT WO 98/03198.

Les chevaux sont vaccinés avec des doses de 100 µg, 250 µg ou 500 µg par plasmide.

Les différents mélanges de plasmides « immunogènes » et du plasmide pJP097 « GM-CSF équin » ainsi obtenus sont co-administrés par voie intramusculaire (seringue + aiguille) dans les muscles du cou ou du poitrail. Dans ce cas, les doses vaccinales sont injectées sous un volume de 2 ml.

Les injections intramusculaires peuvent être aussi réalisées en utilisant un appareil d'injection à jet liquide (sans aiguille) qui propulse une dose de e.g. 0,5 ml. Si nécessaire, plusieurs administrations successives peuvent être faites chez le même animal pour injecter des volumes supérieurs à 0,5 ml. Les tirs successifs sont alors réalisés de manière décalée, de façon que les zones d'injection soient séparées d'environ 1 à 2 centimètres.

Les injections peuvent aussi être réalisées par voie intradermique en utilisant un appareil d'injection à jet liquide (sans aiguille) délivrant une dose de 0,2 ml en 5 points (0,04 ml par point d'injection) (par exemple appareil « PIGJET® » Endoscoptic, Laon, France).

On vaccine typiquement les chevaux par deux injections de mélanges de plasmides selon l'invention réalisées à 4 - 5 semaines d'intervalle.

### Exemple 7 : Formulation des plasmides selon l'invention

Le mélange de plasmides « immunogènes » et du plasmide pJP097 est dilué en tampon TE, en eau physiologique ou en tampon PBS de façon à obtenir une concentration de 1 mg/ml. Une solution de DMRIE-DOPE à 0,75mM est préparée par reprise d'un lyophilisat de DMRIE-DOPE par un volume adapté d'H₂O stérile.

La formation des complexes ADN plasmidique-lipide est réalisée par dilution à parties égales de la solution de DMRIE-DOPE 0,75 mM par la solution d'ADN à 1 mg/ml. La solution d'ADN est introduite progressivement à l'aide d'une aiguille sertie 26G le long de la paroi du flacon contenant la solution de lipide cationique de façon à éviter la formation de mousse. On procède à une agitation douce dès que les deux solutions ont été mélangées. On obtient en final une composition comprenant 0,375 mM de DMRIE-DOPE et 500 µg/ml d'ADN.

Il est souhaitable que l'ensemble des solutions utilisées soient à température ambiante pour l'ensemble des opérations décrites ci-dessus. On laisse la complexation ADN/DMRIE-DOPE se mettre en place à température ambiante pendant 30 minutes avant de procéder à l'immunisation des animaux comme cela est décrit dans l'exemple 6.

## Revendications

1. Adjuvant de vaccin ou stimulant non spécifique de l'immunité pour équidés comprenant un vecteur d'expression *in vivo* contenant une séquence nucléotidique SEQ ID NO :8 codant pour le GM-CSF équin, dans des conditions permettant l'expression chez le cheval d'une protéine GM-CSF équin fonctionnelle.

2. Adjuvant de vaccin ou stimulant non spécifique de l'immunité selon la revendication 1, **caractérisé en ce que** le vecteur est un vecteur viral ou un plasmide.

3. Adjuvant de vaccin ou stimulant non spécifique de l'immunité selon la revendication 2, **caractérisé en ce que** le vecteur viral est choisi dans le groupe consistant en poxvirus, adénovirus et herpèsvirus.

4. Adjuvant de vaccin ou stimulant non spécifique de l'immunité selon la revendication 3, **caractérisé en ce que** l' herpèsvirus est un herpesvirus équin.

5. Adjuvant de vaccin ou stimulant non spécifique de l'immunité selon la revendication 3, **caractérisé en ce que** le poxvirus est choisi dans le groupe consistant en virus de la vaccine, canarypox, fowlpox, swine pox, raccoonpox et camelpox.

6. Composition stimulante de l'immunité pour équidés, comprenant un stimulant non spécifique selon l'une quelconque des revendications 1 à 5, et un excipient ou véhicule acceptable sur le plan vétérinaire.

7. Composition immunogène ou vaccin équin, comprenant un adjuvant de vaccin selon l'une quelconque des revendications 1 à 5, une préparation immunogène ou vaccinale contre un pathogène équin, et un excipient ou véhicule acceptable sur le plan vétérinaire.

8. La composition immunogène ou vaccin équin selon la revendication 7, dans laquelle la préparation immunogène ou vaccinale est choisie dans le groupe consistant en une préparation inactivée, vivante atténuée, de sous-unités et recombinante.

9. Composition immunogène ou vaccin équin comprenant un adjuvant de vaccin selon l'une quelconque des revendications 1 à 5, **caractérisé en ce que** le vecteur est un vecteur d'expression *in vivo* contenant en outre au moins une séquence nucléotidique codant pour au moins un immunogène équin ou un fragment immunologiquement actif d'un immunogène d'un pathogène équin, et un excipient ou véhicule acceptable sur le plan vétérinaire.

10. Composition immunogène ou vaccin équin comprenant un adjuvant de vaccin selon l'une quelconque des revendications 1 à 5, et au moins un vecteur d'expression *in vivo* comprenant au moins une séquence nucléotidique codant pour au moins un immunogène équin ou un fragment immunologiquement actif d'un immunogène d'un pathogène équin, et un excipient ou véhicule acceptable sur le plan vétérinaire.

11. Adjuvant de vaccin ou stimulant non spécifique de l'immunité pour équidés consistant en une protéine équin GM-CSF produite par un système d'expression contenant et exprimant une séquence nucléotidique SEQ ID NO :8.

12. Adjuvant de vaccin ou stimulant non spécifique de l'immunité selon la revendication 11, **caractérisé en ce que** le système d'expression est un vecteur d'expression *in vitro* d'origine virale ou plasmidique.

13. Adjuvant de vaccin ou stimulant non spécifique de l'immunité selon la revendication 12, **caractérisé en ce que** le vecteur d'expression *in vitro* d'origine virale est un bacculovirus.

14. Méthode de production d'un adjuvant de vaccin ou stimulant non spécifique de l'immunité pour équidés, comprenant la transformation ou la transfection d'une cellule *in vitro* par un système d'expression contenant et exprimant une séquence nucléotidique SEQ ID NO :8, et la mise en culture de la cellule dans des conditions permettant l'expression de la protéine GM-CSF équine codée par ladite séquence nucléotidique SEQ ID NO :8.

15. Méthode selon la revendication 14, **caractérisée en ce que** la cellule est choisie dans le groupe consistant en cellule d'insecte, cellule procaryote, cellule eucaryote, cellule de levure et cellule de mammifère.

16. Méthode selon la revendication 15, **caractérisée en ce que** la cellule procaryote est une cellule E. coli.

17. Méthode selon la revendication 15, **caractérisée en ce que** la cellule de levure est une cellule *Saccharomyces cerevisiae*.

18. Méthode selon la revendication 15, **caractérisée en ce que** la cellule de mammifère est une cellule de hamster.

19. Méthode selon la revendication 15, **caractérisée en ce que** la cellule de mammifère est une cellule de chevaux.

20. Composition stimulantes de l'immunité pour équidés, comprenant un stimulant non spécifique selon l'une quelconque des revendications 11 à 13, et un excipient ou véhicule acceptable sur le plan vétérinaire.

21. Composition immunogène ou vaccin équin comprenant un adjuvant de vaccin selon l'une quelconque des revendications 11 à 13, une préparation immunogène
ou vaccinale contre un pathogène équin, et un excipient ou véhicule acceptable sur le plan vétérinaire.

22. La composition immunogène ou vaccin équin selon la revendication 21, dans laquelle la préparation immunogène ou vaccinale est choisie dans le groupe consistant en une préparation inactivée, vivante atténuée, de sous-unités et recombinante.

23. Utilisation d'un stimulant non spécifique de l'immunité selon l'une quelconque des revendications 1, 2, 3, 4, 5, 11, 12, ou 13 pour la fabrication d'une composition stimulante de l'immunité pour équidés.

24. Utilisation d'un adjuvant de vaccin selon l'une quelconque des revendications 1, 2, 3, 4, 5, 11, 12, ou 13 pour la fabrication d'une composition immunogène ou vaccin pour équidés, comprenant en outre un immunogène équin ou une préparation immunogène ou vaccinale.

## Claims

1. Vaccine adjuvant or non specific immunostimulant for an equine comprising an *in vivo* expression vector containing a nucleotide sequence as set forth in SEQ ID NO:8 encoding equine GM-CSF, under conditions enabling the expression in a horse of a functional equine GM-CSF protein.

2. Vaccine adjuvant or non specific immunostimulant according to claim 1, wherein the vector is a viral vector or a plasmid.

3. Vaccine adjuvant or non specific immunostimulant according to claim 2, wherein the viral vector is selected from the group consisting of poxvirus, adenovirus and herpesvirus.

4. Vaccine adjuvant or non specific immunostimulant according to claim 3 wherein the herpesvirus is an equine herpesvirus.

5. Vaccine adjuvant or non specific immunostimulant according to claim 3, wherein the poxvirus is selected from the group consisting of vaccinia virus, canarypox virus, fowlpox virus, swinepox virus, raccoonpox virus and camelpox virus.

6. Composition for immune stimulation in an equine comprising a non specific immunostimulant according to any one of claims 1 to 5, and an acceptable veterinary excipient or vehicle.

7. Equine immunogenic composition or vaccine comprising a vaccine adjuvant according to any one of claims 1 to 5, an immunogen or vaccine preparation against an equine pathogen, and an acceptable veterinary excipient or vehicle.

8. The equine immunogenic composition or vaccine according to claim 7 wherein the immunogen or vaccine preparation is selected in the group consisting of an inactivated preparation, an attenuated live preparation, a subunit preparation and a recombinant preparation.

9. Equine immunogenic composition or vaccine comprising a vaccine adjuvant according to any one of claims 1 to 5, wherein the vector is an *in vivo* expression vector additionally containing at least one a nucleotide sequence encoding at least one equine immunogen or at least one immunologically active fragment of an immunogen of an equine pathogen, and an acceptable veterinary excipient or vehicle.

10. Equine immunogenic composition or vaccine, comprising a vaccine adjuvant according to any one of claims 1 to 5, and at least one *in vivo* expression vector comprising at least one a nucleotide sequence encoding at least one equine immunogen or at least one immunologically active fragment of an immunogen of an equine pathogen, and an acceptable veterinary excipient or vehicle.

11. Vaccine adjuvant or non specific immunostimulant for an equine consisting in an equine GM-CSF protein produced by an expression system containing and expressing the nucleotide sequence as set forth in SEQ ID NO:8.

12. Vaccine adjuvant or non specific immunostimulant according to claim 11 wherein the expression system is an *in vitro* expression vector of viral or plasmidic origin.

13. Vaccine adjuvant or non specific immunostimulant according to claim 12 wherein the *in vitro* viral expression system is a baculovirus.

14. Method for producing a vaccine adjuvant or a non specific immunostimulant for an equine comprising: transforming or transfecting a cell *in vitro* with an expression system containing and expressing a nucleotide sequence as set forth in SEQ ID NO:8 and, culturing said cell under conditions enabling the expression of the equine GM-CSF protein encoded by the nucleotide sequence as set forth in SEQ ID NO:8.

15. Method according to claim 14 wherein the cell is selected from the group consisting of insect cell, prokaryotic cell, eukaryotic cell, yeast cell and mammalian cell.

16. Method according to claim 15 wherein the prokaryotic cell is an *E*. *coli* cell.

17. Method according to claim 15 wherein the yeast cell is a *Saccharomyces cerevisiae* cell.

18. Method according to claim 15 wherein the mammalian cell is a hamster cell.

19. Method according to claim 15 wherein the mammalian cell is an equine cell.

20. Composition for immune stimulation in an equine, comprising a non specific immunostimulant according to any one of claims 11 to 13, and an acceptable veterinary excipient or vehicle.

21. Equine immunogenic composition or vaccine comprising a vaccine adjuvant according to any one of claims 11 to 13, an immunogenic or vaccine preparation against an equine pathogen and an acceptable veterinary excipient or vehicle.

22. The equine immunogenic composition or vaccine according to claim 21, wherein the immunogenic or vaccine preparation is selected from the group consisting of an inactivated preparation, an attenuated live preparation, a subunit preparation, and a recombinant preparation.

23. Use of a non specific immunostimulant according to any one of claims 1, 2, 3, 4, 5, 11, 12, or 13 for the preparation of a composition for immune stimulation in an equine.

24. Use of a vaccine adjuvant according to any one of claims 1 2, 3, 4, 5, 11, 12, or 13 for the preparation of an equine immunogenic composition or vaccine, additionally comprising an equine immunogen or an immunogenic or vaccine preparation.

## Patentansprüche

1. Impfstoff-Adjuvans oder unspezifisches Immunstimulans für Einhufer, das einen *in vivo*-Expressionsvektor umfasst, der eine Pferde-GM-CSF codierende Nucleotidsequenz SEQ ID NO:8 enthält, unter Bedingungen, die die Expression eines funktionellen Pferde-GM-CSF-Proteins in Pferden erlauben.

2. Impfstoff-Adjuvans oder unspezifisches Immunstimulans gemäß Anspruch 1, **dadurch gekennzeichnet, dass** der Vektor ein viraler Vektor oder ein Plasmid ist.

3. Impfstoff-Adjuvans oder unspezifisches Immunstimulans gemäß Anspruch 2, **dadurch gekennzeichnet, dass** der virale Vektor ausgewählt ist aus der Gruppe bestehend aus Pockenvirus, Adenovirus und Herpesvirus.

4. Impfstoff-Adjuvans oder unspezifisches Immunstimulans gemäß Anspruch 3, **dadurch gekennzeichnet, dass** der Herpesvirus ein Pferde-Herpesvirus ist.

5. Impfstoff-Adjuvans oder unspezifisches Immunstimulans gemäß Anspruch 3, **dadurch gekennzeichnet, dass** der Pockenvirus ausgewählt ist aus der Gruppe bestehend aus Kuhpockenvirus, Kanarienpockenvirus, Geflügelpockenvirus, Schweinepockenvirus, Waschbärpockenvirus und Kamelpockenvirus.

6. Immunstimulierende Zusammensetzung für Einhufer, die ein unspezifisches Stimulans gemäß einem der Ansprüche 1 bis 5 und einen verterinärmedizinisch verträglichen Excipienten oder Träger umfasst.

7. Immunogene Zusammensetzung oder Pferdeimpfstoff, die/der ein Impfstoff-Adjuvans gemäß einem der Ansprüche 1 bis 5, ein immunogenes Präparat oder ein Impfstoffpräparat gegen einen Pferdekrankheitserreger und einen verterinärmedizinisch verträglichen Excipienten oder Träger umfasst.

8. Immunogene Zusammensetzung oder Pferdeimpfstoff gemäß Anspruch 7, wobei das immunogene Präparat oder das Impfstoffpräparat ausgewählt ist aus der Gruppe bestehend aus einem inaktivierten Präparat, einem lebend attenuierten Präparat, einem Präparat aus Untereinheiten und einem rekombinanten Präparat.

9. Immunogene Zusammensetzung oder Pferdeimpfstoff, umfassend ein Impfstoff-Adjuvans gemäß einem der Ansprüche 1 bis 5, **dadurch gekennzeichnet, dass** der Vektor ein *in vivo*-Expressionsvektor ist, welcher ferner mindestens eine Nucleotidsequenz umfasst, die mindestens ein Pferde-Immunogen oder ein immunologisch aktives Fragment eines Immunogens eines Pferde-Krankheitserregers codiert, und einen verterinärmedizinisch verträglichen Excipienten oder Träger.

10. Immunogene Zusammensetzung oder Pferdeimpfstoff, umfassend ein Impfstoff-Adjuvans gemäß einem der Ansprüche 1 bis 5, und mindestens einen *in vivo*-Expressionsvektor, welcher mindestens eine Nucleotidsequenz umfasst, die mindestens ein Pferde-Immunogen oder ein immunologisch aktives Fragment eines Immunogens eines Pferde-Krankheitserregers codiert, und einen verterinärmedizinisch verträglichen Excipienten oder Träger.

11. Impfstoff-Adjuvans oder unspezifisches Immunstimulans für Einhufer, die aus einem Pferde-GM-CSF-Protein besteht, welches durch ein Expressionssystem produziert wird, das eine Nucleotidsequenz SEQ ID NO:8 enthält und exprimiert.

12. Impfstoff-Adjuvans oder unspezifisches Immunstimulans gemäß Anspruch 11, **dadurch gekennzeichnet, dass** das Expressionssystem ein *in vitro-*Expressionsvektor ist, dessen Ursprung viral oder ein Plasmid ist.

13. Impfstoff-Adjuvans oder unspezifisches Immunstimulans gemäß Anspruch 12, **dadurch gekennzeichnet, dass** der *in vitro*-Expressionsvektor viralen Ursprungs ein Bacculovirus ist.

14. Verfahren zur Herstellung eines Impfstoff-Adjuvans oder eines unspezifischen Immunstimulans für Einhufer, umfassend die Transformation oder die Transfektion einer Zelle *in vitro* mit einem Expressionssystem, das eine Nucleotidsequenz SEQ ID NO:8 enthält und exprimiert, und das Züchten der Zelle unter Bedingungen, die die Expression des von der Nucleotidsequenz SEQ ID NO:8 codierten Pferde-GM-CSF-Proteins erlauben.

15. Verfahren gemäß Anspruch 14, **dadurch gekennzeichnet, dass** die Zelle ausgewählt ist aus der Gruppe bestehend aus Insektenzelle, prokaryontischer Zelle, eukaryontischer Zelle, Hefezelle und Säugerzelle.

16. Verfahren gemäß Anspruch 15, **dadurch gekennzeichnet, dass** die prokaryontische Zelle eine E. coli-Zelle ist.

17. Verfahren gemäß Anspruch 15, **dadurch gekennzeichnet, dass** die Hefezelle eine *Saccharomyces cerevisiae*-Zelle ist.

18. Verfahren gemäß Anspruch 15, **dadurch gekennzeichnet, dass** die Säugerzelle eine Hamsterzelle ist.

19. Verfahren gemäß Anspruch 15, **dadurch gekennzeichnet, dass** die Säugerzelle eine Pferdezelle ist.

20. Immunstimulierende Zusammensetzung für Einhufer, die ein unspezifisches Stimulans gemäß einem der Ansprüche 11 bis 13 und einen verterinärmedizinisch verträglichen Excipienten oder Träger umfasst.

21. Immunogene Zusammensetzung oder Pferdeimpfstoff, die/der ein Impfstoff-Adjuvans gemäß einem der Ansprüche 11 bis 13, ein immunogenes Präparat oder ein Impfstoffpräparat gegen einen Pferdekrankheitserreger und einen verterinärmedizinisch verträglichen Excipienten oder Träger umfasst.

22. Immunogene Zusammensetzung oder Pferdeimpfstoff gemäß Anspruch 21, wobei das immunogene Präparat oder das Impfstoffpräparat ausgewählt ist aus der Gruppe bestehend aus einem inaktivierten Präparat, einem lebend attenuierten Präparat, einem Präparat aus Untereinheiten und einem rekombinanten Präparat.

23. Verwendung eines unspezifischen Immunstimulans gemäß einem der Ansprüche 1, 2, 3, 4, 5, 11, 12 oder 13 für die Herstellung einer immunstimulierenden Zusammensetzung für Einhufer.

24. Verwendung eines Impfstoff-Adjuvans gemäß einem der Ansprüche 1, 2, 3, 4, 5, 11, 12 oder 13 für die Herstellung einer immunogenen Zusammensetzung oder eines Impfstoffs für Einhufer, die/der ferner ein Pferdeimmunogen oder ein immunogenes Präparat oder ein Impfstoffpräparat umfasst.


### LISTE DE SEQUENCES

<110> MERIAL
<120> Equine GM-CSF
<130> GM-CSF équin
<140> numéro brevet
   <141> date dépôt brevet
<160> 9
<170> PatentIn Ver. 2.1
<210> 1
   <211> 20
   <212> ADN
   <213> Artificial Sequence
<220>
   <223> Description of Artificial Sequence:
   oligonucléotide
<400> 1
   tgggcactgt ggyctgcagc 20
<210> 2
   <211> 17
   <212> ADN
   <213> Artificial Sequence
<220>
<223> Description of Artificial Sequence:
   oligonucléotide
<400> 2
   agcatgtgra tgccatc 17
<210> 3
   <211> 21
   <212> ADN
   <213> Artificial Sequence
<220>
   <223> Description of Artificial Sequence:
   oligonucléotide
<400> 3
   agctcccagg gctagctcct a 21
<210> 4
   <211> 21
   <212> ADN
   <213> Artificial Sequence
<220>
   <223> Description of Artificial Sequence:
   oligonucléotide
<400> 4
   ccctgtttgt acagcttcag g 21
<210> 5
   <211> 21
   <212> ADN
   <213> Artificial Sequence
<220>
   <223> Description of Artificial Sequence:
   oligonucléotide
<400> 5
   tgttattcag aaggctcagg g 21
<210> 6
   <211> 44
   <212> ADN
   <213> Artificial Sequence
<220>
   <223> Description of Artificial Sequence:
   oligonucléotide
<400> 6
   catcatcatg tcgacgccac catgtggctg cagaacctgc ttct 44
<210> 7
   <211> 41
   <212> ADN
   <213> Artificial Sequence
<220>
   <223> Description of Artificial Sequence:
   oligonucléotide
<400> 7
   catcatcatg cggccgctac ttcugggctg ctggcttcca g 41
<210> 8
   <211> 435
   <212> ADN
   <213> Equus sp.
<400> 8
   atgtggctgc agaacctgct tcttctgggc actgtggttt acagcatgcc cgcacccacc 60
   cgccaaccca gccctgtcac tcggccctgg cagcatgtgg atgccatcaa ggaggccctg 120
   agccttctga acaacagtag tgacactgct gctatcatga atgaaacagt agaagtcgtc 180
   tctgaaacgt ttgacgccga ggagctgaca tgcctgcaga ctcgcctgaa gctgtacaaa 240
   cagggcttgc ggggcagcct catcaagctc gaaggcccct tgaccatgat ggccagccac 300
   tacaagcagc actgcccccc caccctggaa acttcctgtg caacccagat gatcaccttc 360
   aaaagtttca aaaagaacct gaaggatttt ctgtttgaga tcccgtttga ctgctggaag 420
   ccagcccaga agtaa                                                  435
<210> 9
   <211> 144
   <212> PRT
   <213> Equus sp.
<400> 9
   Met Trp Leu Gln Asn Leu Leu Leu Leu Gly Thr Val Val Tyr Ser Met
    1               5                  10                  15
   Pro Ala Pro Thr Arg Gln Pro Ser Pro Val Thr Arg Pro Trp Gln His
               20                  25                  30
   Val Asp Ala Ile Lys Glu Ala Leu Ser Leu Leu Asn Asn Ser Ser Asp
           35                  40                  45
   Thr Ala Ala Ile Met Asn Glu Thr Val Glu Val Val Ser Glu Thr Phe
       50                  55                  60
   Asp Ala Glu Glu Leu Thr Cys Leu Gln Thr Arg Leu Lys Leu Tyr Lys
   65                  70                  75                  80
   Gln Gly Leu Arg Gly Ser Leu Ile Lys Leu Glu Gly Pro Leu Thr Met
                   85                  90                  95
   Met Ala Ser His Tyr Lys Gln His Cys Pro Pro Thr Leu Glu Thr Ser
               100                 105                 110
   Cys Ala Thr Gln Met Ile Thr Phe Lys Ser Phe Lys Lys Asn Leu Lys
           115                 120                 125
   Asp Phe Leu Phe Glu Ile Pro Phe Asp Cys Trp Lys Pro Ala Gln Lys
       130                 135                 140